(19) Europäisches Patentamt
European Patent Office
Office européen des brevets

(11) **EP 4 023 335 A1**

(12) **EUROPÄISCHE PATENTANMELDUNG**

(43) Veröffentlichungstag:
06.07.2022 Patentblatt 2022/27

(21) Anmeldenummer: 21214724.3

(22) Anmeldetag: 15.12.2021

(51) Internationale Patentklassifikation (IPC):
**B01J 38/04** (2006.01)        **C07C 15/00** (2006.01)
**B01J 23/96** (2006.01)        **B01J 23/94** (2006.01)

(52) Gemeinsame Patentklassifikation (CPC):
(C-Sets verfügbar)
**B01J 38/04; B01J 23/94; B01J 23/96;**
**C07C 67/303;** C07C 2601/14        (Forts.)

(84) Benannte Vertragsstaaten:
**AL AT BE BG CH CY CZ DE DK EE ES FI FR GB**
**GR HR HU IE IS IT LI LT LU LV MC MK MT NL NO**
**PL PT RO RS SE SI SK SM TR**
Benannte Erstreckungsstaaten:
**BA ME**
Benannte Validierungsstaaten:
**KH MA MD TN**

(30) Priorität: **18.12.2020 EP 20215552**

(71) Anmelder: **Evonik Operations GmbH**
**45128 Essen (DE)**

(72) Erfinder:
• **Anton, Johan**
**46284 Dorsten (DE)**

• **Grass, Michael**
**45721 Haltern am See (DE)**
• **Kraft, Johannes**
**53859 Niederkassel (DE)**
• **Schneider, Thomas**
**46514 Schermbeck (DE)**
• **Ziomek, Grzegorz**
**45665 Recklinghausen (DE)**

(74) Vertreter: **Evonik Patent Association**
**c/o Evonik Industries AG**
**IP Management**
**Bau 1042A/PB 15**
**Paul-Baumann-Straße 1**
**45772 Marl (DE)**

(54) **VERFAHREN ZUR REGENERIERUNG VON HYDRIERKATALYSATOREN**

(57) Gegenstand der Erfindung ist ein Verfahren zur Regenerierung eines für die Hydrierung eines Aromaten eingesetzten Katalysators, welches aus mehreren Schritten besteht. Zunächst wird mit Stickstoff gespült, dann schrittweise Luft zudosiert und anschließend die Zugabe von Stickstoff beendet bis nur noch Luft vorliegt.

EP 4 023 335 A1

**(Forts. nächste Seite)**

(52) Gemeinsame Patentklassifikation (CPC): (Forts.)

C-Sets
**C07C 67/303, C07C 69/75**

**Beschreibung**

Die vorliegende Erfindung betrifft ein Verfahren zur Regenerierung eines für die Hydrierung eines Aromaten eingesetzten Katalysators, welches aus mehreren Schritten besteht. Zunächst wird mit Stickstoff gespült, dann schrittweise Luft zudosiert und anschließend die Zugabe von Stickstoff beendet bis nur noch Luft vorliegt.

Die Hydrierung von Aromaten, insbesondere von aromatischen Estern, bei der der aromatische Ring hydriert wird, ist bekannt und wird auch als Kernhydrierung bezeichnet. Bei einer solchen Hydrierung werden beispielsweise Übergangsmetall-enthaltende Katalysatoren eingesetzt. Geeignete Katalysatoren sind dem Fachmann bekannt. Entsprechende Hydrierverfahren werden auch großindustriell eingesetzt.

Mit fortschreitender Dauer der Hydrierung können die eingesetzten Katalysatoren an Aktivität verlieren, sei es durch Blockieren, Verlust oder Vergiften der aktiven Zentren des Katalysators. Ist der Aktivitätsverlust zu hoch und/oder lässt sich das Hydrierverfahren nicht mehr ausreichend wirtschaftlich betreiben, muss die Aktivität des Katalysators gesteigert werden. Dies geschieht mittels eines Regenerierverfahrens, bei dem der Katalysator von Ablagerungen und Anhaftungen befreit wird. Möglich ist dabei eine Kalzination des Katalysators, bei dem die Ablagerungen bei hohen Temperaturen (> 200 °C) an Luft oder in einer Inertgasatmosphäre entfernt werden, was jedoch für bestimmte Katalysatoren oder auch rein energetisch aufgrund der hohen Temperaturen problematisch sein kann.

Katalysatoren können aber auch durch Überleiten eines Gasstroms regeneriert werden, durch den die Ablagerungen mitgerissen und dadurch entfernt werden. Ein solches Verfahren zur Regenerierung von Rutheniumkatalysatoren wird beispielsweise in der WO 2008/015103 A1 beschrieben. Die dort offenbarte Regenerierung zeichnet sich dadurch aus, dass der Katalysator mit einem Inertgas gespült wird, bis der Katalysator seine Aktivität teilweise oder sogar vollständig wiedererlangt hat. Als Grund für die dort anhand der Benzolhydrierung beschriebene regenerierende Wirkung wird die Entfernung von Wasser, d. h. das Trocknen des Katalysators genannt.

Das bekannte Verfahren weist jedoch das Problem auf, dass der Regeneriereffekt zu gering sein kann, insbesondere dann, wenn nicht nur Wasser vom Katalysator entfernt werden muss oder sich nicht nur Wasser, sondern auch andere, die Hydrieraktivität senkende, Substanzen auf dem Katalysator befinden.

Demzufolge bestand die Aufgabe der vorliegenden Erfindung darin, Verfahren zur Regenerierung eines für die Kernhydrierung eines Aromaten, insbesondere eines aromatischen Esters, eingesetzten Katalysators bereitzustellen, mit dem eine akzeptable Aktivität schneller und besser erreicht werden kann.

Die Aufgabe wird durch das in Anspruch 1 genannte Verfahren gelöst. Bevorzugte Ausführungsformen sind in den Unteransprüchen angegeben. Das erfindungsgemäße Verfahren ist ein Verfahren zur Regenerierung eines für die Kernhydrierung eines Aromaten, vorzugsweise zur Kernhydrierung eines aromatischen Esters, eingesetzten Katalysators in mindestens einem Reaktor, wobei das Verfahren die folgenden Schritte umfasst:

- Spülen des mindestens einen Reaktors, in dem der eingesetzte Katalysator vorliegt, mit einem Inertgas, wobei das Spülen mit einem Inertgas, vorzugsweise mit Stickstoff bei einer Temperatur von 50 bis 100 °C gestartet wird; gefolgt von
- einer schrittweisen Zugabe von Luft zum Inertgas während des Spülens des Reaktors bis der Anteil der Luft an dem Gesamtvolumen zwischen 10 und 90 Vol.-% liegt; und anschließend
- einer Reduzierung der Inertgasmenge, vorzugsweise der Stickstoffmenge, während des Spülens des Reaktors bis der Reaktor nur noch von Luft durchströmt wird,

wobei die Temperatur im Reaktor während der Regenerierung maximal 10°C höher ist als die Temperatur am Zulauf des Reaktors für den Aromaten, vorzugsweise den aromatischen Ester, bei der Hydrierung des Aromaten, vorzugsweise des aromatischen Esters,

wobei der Katalysator mindestens ein Übergangsmetall, welches aus der Gruppe, bestehend aus Eisen, Ruthenium, Nickel, Rhodium, Platin, Palladium oder Mischungen davon, ausgewählt wird auf einem Trägermaterial, welches aus der Gruppe, bestehend aus Aktivkohle, Siliciumcarbid, Aluminiumoxid, Siliciumdioxid, Alumosilikat, Titandioxid, Zirkoniumdioxid, Magnesiumoxid, Zinkoxid oder Mischungen davon, ausgewählt wird umfasst.

Durch das erfindungsgemäße Verfahren kann eine vorteilhafte Regenerierung des Hydrierkatalysators erreicht werden, mit dem die Katalysatoraktivität sehr einfach und schnell verbessert wird. Die hier beschriebene Regenerierung ermöglicht also, dass die Kernhydrierung, wodurch die eigentlichen Wertprodukte hergestellt werden, anschließend bei gesteigerten Umsätzen wieder in Betrieb genommen werden und somit effektiver durchgeführt werden kann. Gleichzeitig verhindert die eher geringe Sauerstoffkonzentration bzw. der entsprechend gesteuerte Verfahrensablauf, dass es durch Oxidationsprozesse zu stark (gegebenenfalls auch nur lokal) erhöhten Temperaturen im Reaktor kommt, die den Katalysator schädigen oder im schlimmsten Fall zu Schäden am Reaktor führen können.

Die hier beschriebene Kernhydrierung von Aromaten, insbesondere von aromatischen Estern, wird üblicherweise in mindestens einem Reaktor durchgeführt, d. h. die Kernhydrierung kann in einem oder mehreren Reaktoren erfolgen, die jeweils einen geeigneten Katalysator enthalten. Die erfindungsgemäße Regenerierung des eingesetzten Katalysators kann in jedem der vorliegenden Reaktoren erfolgen. Die Regenerierung kann bei Vorliegen von mehreren Reaktoren für jeden Reaktor zeitlich versetzt oder gleichzeitig erfolgen, wobei eine gleichzeitige Regenerierung aller Katalysatoren in allen vorliegenden Reaktoren bevorzugt ist. Erfindungsgemäßer Vorteil ist also, dass der Katalysator für die Regenerierung nicht aus dem Reaktor herausgeholt werden muss, sondern in dem Reaktor verbleiben kann, in dem auch die Kernhydrierung stattfindet.

Erfindungsgemäß eignet sich das Verfahren für alle bei der Kernhydrierung von Aromaten, insbesondere von aromatischen Estern eingesetzten Katalysatoren. Der Katalysator umfasst mindestens ein Übergangsmetall auf einem Trägermaterial oder besteht aus mindestens einem Übergangsmetall auf einem Trägermaterial. Geeignete Katalysatoren sind dem Fachmann auch geläufig und beispielsweise der WO 03/103830 A1 zu entnehmen.

Das Übergangsmetall des zu regenerierenden Katalysators ist ein Metall, welches aus der Gruppe, bestehend aus Eisen, Ruthenium, Nickel, Rhodium, Platin, Palladium oder Mischungen davon, ausgewählt wird. Ruthenium ist das in der vorliegenden Erfindung besonders bevorzugte Übergangsmetall des eingesetzten Katalysators. Der Gehalt an Übergangsmetall im zu regenerierenden Katalysator beträgt im Allgemeinen 0,1 bis 30 Massen-%. Der Rutheniumgehalt, berechnet als Metall, liegt vorzugsweise im Bereich von 0,1 bis 10 Massen-%, insbesondere im Bereich von 0,3 bis 5 Massen-%, ganz besonders im Bereich zwischen 0,4 und 2,5 Massen-%.

Das Trägermaterial, auf dem sich das Übergangsmetall befindet, wird aus der Gruppe, bestehend aus Aktivkohle, Siliciumcarbid, Aluminiumoxid, Siliciumdioxid, Alumosilikat, Titandioxid, Zirkoniumdioxid, Magnesiumoxid, Zinkoxid oder Mischungen davon, ausgewählt. Bevorzugte Trägermaterialien sind Aluminiumoxid, Siliciumdioxid, Titandioxid und Mischung davon. Zusätzlich können diese Trägermaterialien Alkalimetalle, Erdalkalimetalle und/oder Schwefel enthalten. In einer besonders bevorzugten Ausführungsform der vorliegenden Erfindung ist der zu regenerierende Katalysator ein Schalenkatalysator.

Das erfindungsgemäße Verfahren zur Regenerierung eines für die Kernhydrierung eines Aromaten, vorzugsweise zur Kernhydrierung eines aromatischen Esters, eingesetzten Katalysators umfasst drei Schritte. Diese Schritte erfolgen in dem mindestens einen Reaktor, werden demzufolge ohne Ausbau des Katalysators durchgeführt. Bei den drei Schritten werden Gasströme mit je nach dem vorliegenden Schritt unterschiedlicher Zusammensetzung durch die Reaktoren, in denen sich die Katalysatoren befinden, geleitet. Dadurch wird der Katalysator von den möglichen Ablagerungen und Anhaftungen befreit.

Im ersten Schritt wird der Reaktor bzw. die Reaktoren mit einem Inertgas durchströmt und dadurch gespült. Das Inertgas kann dabei aus der Gruppe, bestehend aus Stickstoff, Helium, Neon, Argon, Kohlendioxid und Mischungen daraus, ausgewählt werden. Bevorzugt werden Stickstoff oder Kohlendioxid eingesetzt, da diese am einfachsten und günstigsten verfügbar sind. Besonders bevorzugt ist Stickstoff das Inertgas für das erfindungsgemäße Verfahren.

Die Temperatur ist ein wichtiges Merkmal der vorliegenden Erfindung. So darf die Temperatur im Reaktor während der Regenerierung maximal 10°C höher sein als die Temperatur am Zulauf des Reaktors für den aromatischen Ester bei der Hydrierung des aromatischen Esters. Ein Abbrennen des Katalysators oder eine Kalzinierung des Katalysators, was durch einen durch die Zugabe von Luftsauerstoff bedingten, ggf. extremen Temperaturanstieg hervorgerufen werden kann, soll nach der erfindungsgemäßen Lehre somit verhindert werden. Die Temperatur im Reaktor kann erfindungsgemäß an jeder beliebigen Position innerhalb des Reaktors gemessen werden. Der erste Schritt, also das Spülen mit einem Inertgas, vorzugsweise das Spülen mit Stickstoff, wird bei einer Temperatur von 50 bis 100 °C gestartet. Da die Temperatur bei der vor der Regenerierung stattfindenden Kernhydrierung höher als 100 °C sein kann, kann es notwendig sein, dass der Reaktor vor der Regenerierung gekühlt wird, beispielsweise durch einfaches Stehenlassen oder aktives Kühlen mit Durchfahren des Hydrierprodukts durch einen Kühler.

Es ist bevorzugt, dass die Temperatur während der Regenerierung mit der Zeit absinkt. Durch die nachfolgenden Schritte kann es jedoch zu temporären, kurzzeitigen Temperaturanstiegen kommen. Über die Zeit sollte sich trotzdem ein Absinken der Temperatur feststellen lassen, da die Reaktoren oder die Gasströme nicht gezielt erwärmt werden.

Vor dem ersten Schritt, dem Spülen des Reaktors mit Inertgas, vorzugsweise Stickstoff, und nach Abstellen der Hydrierung können weitere Schritte durchgeführt werden. Vorzugsweise werden die folgenden Schritte durchgeführt:

• Entspannen des mindestens einen Reaktors auf einen Druck von weniger als 10 bar, vorzugsweise weniger als 7,5 bar, besonders bevorzugt weniger als 5 bar;

• Verdrängen des restlichen Wasserstoffs aus dem mindestens einen Reaktor durch Aufdrücken eines Inertgases, vorzugsweise von Stickstoff auf einen Druck von mehr als 10 bar, vorzugsweise mehr als 12,5 bar, besonders bevorzugt mehr als 15 bar und anschließendes Entspannen des mindestens einen Reaktors auf einen Druck von weniger als 10 bar, vorzugsweise weniger als 7,5 bar, besonders bevorzugt weniger als 5 bar.

Der Ablauf aus Aufdrücken eines Inertgases, vorzugsweise von Stickstoff und das anschließende Entspannen werden in einer bevorzugten Ausführungsform mindestens zweimal durchgeführt.

Anschließend erfolgt dann der erste Schritt des erfindungsgemäßen Regenerierverfahrens, also das Spülen mit Inertgas.

Im zweiten Schritt des erfindungsgemäßen Regenerierverfahrens wird während des Spülens des Reaktors oder der Reaktoren schrittweise Luft zum Inertgas zugegeben bis der Anteil der Luft an dem Gesamtvolumen zwischen 10 und 90 Vol.-%, vorzugsweise zwischen 20 und 80 Vol.-%, besonders bevorzugt zwischen 20 und 60 Vol.-% liegt. Die schrittweise Zugabe von Luft erfolgt vorzugsweise in mindestens 3 Schritten, wobei erst mit dem letzten Schritt der oben genannte Anteil an Luft vorliegt. Durch die Zugabe von Luft wird Sauerstoff in das System geleitet, was zu Oxidationsreaktionen und damit lokal zu einem Anstieg der Temperatur führen kann. Es ist daher ratsam, die Temperatur in dem mindestens einen Reaktor zu überwachen, beispielsweise mit geeigneten Messfühlern. Vorteilhaft kann es sein, dass mehrere Messfühler in dem mindestens einen Reaktor vorhanden sind, damit lokal auftretende Temperatursteigerungen auch festgestellt werden können.

In einer bevorzugten Ausführungsform ist die Temperatur im zweiten Schritt, besonders bevorzugt auch temporär, während eines Schrittes der schrittweisen Zugabe von Luft maximal 20 °C, besonders bevorzugt maximal 15 °C höher ist als zu Beginn des gleichen jeweiligen Schrittes der schrittweisen Zugabe von Luft. Die Zugabe von Luft erfolgt dabei kontrolliert, wodurch ein Abbrennen des Katalysators und/oder eine übermäßige Oxidation am Katalysator verhindert wird. Sollte es dennoch, auch lokal, zu einer höheren Temperatur kommen, ist es ratsam, dass gegensteuernde Maßnahmen eingeleitet werden. Erfindungsgemäß bevorzugt wird die (weitere) Zugabe von Luft zumindest zeitweise abgestellt, wenn die Temperatur während eines Schrittes der schrittweisen Zugabe von Luft 20 °C, vorzugsweise 15 °C oder mehr über der Temperatur zu Beginn des gleichen jeweiligen Schrittes der schrittweisen Zugabe von Luft liegt. Es ist auch möglich, dass zusätzlich Inertgas, vorzugsweise Stickstoff, während des Abstellens der weiteren Zugabe von Luft zudosiert wird.

Der dritte Schritt des erfindungsgemäßen Regenerierverfahrens zeichnet sich dadurch aus, dass die Inertgasmenge, vorzugsweise die Stickstoffmenge, während des Spülens des Reaktors schrittweise reduziert wird, bis der Reaktor nur noch von Luft durchströmt wird. Dieser Schritt kann sowohl erst nach Beendigung des zweiten Schrittes als auch teilweise oder vollständig während der Durchführung des zweiten Schrittes erfolgen. Sollte es hier zu einer (lokalen) Erhöhung der Temperatur kommen, wäre ein zeitweises Abbrechen der Reduzierung der Inertgasmenge oder sogar ein weiteres Zudosieren von Inertgas, vorzugsweise Stickstoff, möglich. Der dritte Schritt, also die Durchströmung mit Luft, erfolgt vorzugsweise bei einer Temperatur von bis zu 70 °C, wenn die Zugabe von Inertgas vollständig beendet wurde. Bevorzugt wird die Temperatur mit der Zeit weiter absinken, vorzugsweise auf Umgebungstemperatur, d.h. auf eine Temperatur in einem Bereich von 20 - 35 °C.

Es hängt von verschiedenen Faktoren ab, wie lange die vorliegend beschriebene Regenerierung durchgeführt werden muss, um die Katalysatoraktivität in ausreichendem Maße zu verbessern. Beispiele für diese Faktoren sind die Art und Menge der Verunreinigungen oder die Beschaffenheit der Reaktoren (Größe, Durchmesser). Die Dauer der gesamten Regenerierung, also die Durchführung aller Schritte, beträgt vorzugsweise mindestens 24 Stunden, besonders bevorzugt mindestens 3 Tage. Bei kürzeren Zeiten kann zwar die Anlage schneller wieder in Betrieb genommen werden. Die Regenerierung erfolgt dann jedoch nicht so umfänglich und es kann notwendig sein, dass zeitnah eine erneute Regenerierung erforderlich ist.

Die Gasströme in den einzelnen Schritten des erfindungsgemäßen Verfahrens werden vorzugsweise mit einem Volumenstrom von mindestens 2 m³ pro m³ Katalysator und pro Stunde durch den Reaktor bzw. über den Katalysator geleitet. Solche Volumenströme ermöglichen eine vergleichsweise schnelle Regeneration. Der Gasstrom bei den einzelnen Schritten der Regenerierung kann in gleicher oder umgekehrter Strömungsrichtung, bezogen auf die Strömungsrichtung bei der Hydrierung, über den Katalysator geleitet werden, vorzugsweise geschieht dies in gleicher Strömungsrichtung.

Die erfindungsgemäße Regenerierung erfolgt bei einem in der Kernhydrierung eines Aromaten eingesetzten Katalysator. In einer bevorzugten Ausführungsform erfolgt die erfindungsgemäße Regenerierung bei einem in der Kernhydrierung eines aromatischen Esters eingesetzten Katalysators. Der aromatische Ester ist vorzugsweise ein Ester einer Benzolcarbonsäure, ein Ester einer Benzoldicarbonsäure, ein Ester einer Benzoltricarbonsäure oder ein Ester einer Benzoltetracarbonsäure, vorzugsweise ein Ester der Benzoldicarbonsäure oder der Benzoltricarbonsäure. Dazu zählen Phthalate, Isophthalate, Terephthalate und Trimellitate. Bevorzugt sind Ester der Benzoldicarbonsäure, also Phthalate, Isophthalate und Terephthalate. Davon sind besonders die C8- bis C10-Alkylester, insbesondere die C8- bis C10-Alkylester der Phthalsäure (z. B. Dioctylphthalat, Diethylhexylphthalat, Diisononylphthalat, Dipropylheptylphthalat) oder die C8- bis C10-Alkylester der Terephthalsäure (z. B. Dioctylterephthalat, Diethylhexylterephthalat, Diisononylterephthalat, Dipropylheptylterephthalat) bevorzugt.

Die Kernhydrierung wird bevorzugt in flüssiger Phase durchgeführt. Die Kernhydrierung kann an suspendierten oder stückigen im Festbett angeordneten Katalysatoren kontinuierlich oder diskontinuierlich durchgeführt werden. Im erfindungsgemäßen Verfahren wird eine kontinuierliche Kernhydrierung an einem im Festbett angeordnetem Katalysator, bei dem sich unter Reaktionsbedingungen die Produkt/Eduktphase hauptsächlich im flüssigen Zustand befindet, bevorzugt. Vorzugsweise wird/werden der/die Reaktor(en) als Rieselbettreaktor betrieben, der/die vollständig oder teilweise geflutet sein kann/können.

Wenn die Kernhydrierung kontinuierlich an einem im Festbett angeordneten Katalysator durchgeführt wird, ist es zweckmäßig, den Katalysator vor der erstmaligen Durchführung der Kernhydrierung in die aktive Form zu überführen. Nach einer Regenerierung ist eine solche Aktivierung nicht unbedingt erforderlich. Die Aktivierung kann durch Reduktion des Katalysators mit Wasserstoff-haltigen Gasen nach einem Temperaturprogramm erfolgen. Dabei kann die Reduktion gegebenenfalls in Gegenwart einer flüssigen Phase, die über den Katalysator rieselt, durchgeführt werden. Als flüssige Phase kann dabei ein Lösemittel oder das Hydrierprodukt eingesetzt werden. Bei entsprechenden Hydrierbedingungen ist auch ein Verzicht auf eine derartige Aktivierung möglich, da diese auch unter Reaktionsbedingungen erfolgt.

Für die Kernhydrierung können unterschiedliche Verfahrensvarianten gewählt werden. Es kann adiabatisch, polytrop oder praktisch isotherm, d. h. mit einem Temperaturanstieg von typischerweise kleiner als 10 °C, ein- oder mehrstufig durchgeführt werden. Im letzteren Fall kann man alle Reaktoren, zweckmäßig Rohrreaktoren, adiabatisch oder praktisch isotherm sowie einen oder mehrere adiabatisch und die anderen praktisch isotherm betreiben. Weiterhin ist es möglich, die aromatischen Polycarbonsäureester im geraden Durchgang oder unter Produktrückführung zu hydrieren. Es ist auch möglich, dass zwei Reaktoren vorhanden sind, wobei der erste Reaktor unter Produktrückführung und der zweite Reaktor im geraden Durchgang betrieben wird. Möglich ist auch eine Durchführung der Kernhydrierung als Trickle-Bed. Der Reaktor kann dabei auch teilweise oder vollständig geflutet sein. Weiterhin können auch mehr als zwei Reaktoren verwendet werden. Besonders bevorzugt wäre auch in diesem Fall, dass alle Reaktoren bis auf den letzten unter Produktrückführung (sog. Schlaufenreaktor) betrieben werden und nur der letzte im geraden Durchgang.

Die Kernhydrierung kann in der Flüssig/Gas-Mischphase oder in der Flüssigphase in Dreiphasenreaktoren im Gleichstrom durchgeführt werden, wobei das Hydriergas in an sich bekannter Weise im flüssigen Edukt/Produktstrom verteilt wird. Im Interesse einer gleichmäßigen Flüssigkeitsverteilung, einer verbesserten Reaktionswärmeabfuhr und einer hohen Raum-Zeit-Ausbeute werden die Reaktoren vorzugsweise mit hohen Flüssigkeitsbelastungen von 15 bis 120, insbesondere von 25 bis 80 $m^3$ pro $m^2$ Querschnitt des leeren Reaktors und Stunde betrieben. Wird ein Reaktor im geraden Durchgang betrieben, so kann die spezifische Katalysatorbelastung (LHSV) Werte zwischen 0,1 und 10 $h^{-1}$ annehmen.

Die Kernhydrierung kann in Ab- oder vorzugsweise in Anwesenheit eines Lösungsmittels durchgeführt werden. Als Lösemittel können alle Flüssigkeiten eingesetzt werden, die mit dem Edukt und Produkt eine homogene Lösung bilden, sich unter Hydrierbedingungen inert verhalten und sich leicht vom Produkt abtrennen lassen. Das Lösemittel kann auch ein Gemisch mehrerer Stoffe sein und gegebenenfalls Wasser enthalten.

Beispielsweise können folgende Stoffe als Lösemittel eingesetzt werden: Geradkettige oder cyclische Ether, wie beispielsweise Tetrahydrofuran oder Dioxan sowie aliphatische Alkohole, in denen der Alkylrest 1 bis 13 Kohlenstoffatome aufweist. Bevorzugt verwendbare Alkohole sind beispielsweise Isopropanol, n-Butanol, Isobutanol, n-Pentanol, 2-Ethylhexanol, Nonanole, technische Nonanolgemische, Decanol, technische Decanolgemische, Tridecanole.

Bei Einsatz von Alkoholen als Lösemittel kann es zweckmäßig sein, denjenigen Alkohol oder dasjenige Alkoholgemisch zu verwenden, das bei der Verseifung des Produkts entstehen würde. Dadurch ist die Nebenproduktbildung durch Umesterung ausgeschlossen. Ein weiteres bevorzugtes Lösemittel ist das Hydrierprodukt selbst.

Durch die Verwendung eines Lösemittels kann die Aromatenkonzentration im Reaktorzulauf begrenzt werden, wodurch eine bessere Temperaturkontrolle im Reaktor erreicht werden kann. Dies kann eine Minimierung von Nebenreaktionen und somit eine Erhöhung der Produktausbeute zu Folge haben. Bevorzugt liegt der Aromatengehalt im Reaktorzulauf zwischen 1 und 35 %, insbesondere zwischen 5 und 25 %. Der gewünschte Konzentrationsbereich kann bei Reaktoren, die in Schlaufenfahrweise betrieben werden, durch die Zirkulationsrate (Mengenverhältnis von rückgeführtem Hydrieraustrag zu Edukt) eingestellt werden.

Die Kernhydrierung kann in einem Druckbereich von 20 bis 300 bar, vorzugsweise 40 bis 200 bar durchgeführt werden. Die Hydriertemperaturen liegen vorzugsweise im Bereich von 60 bis 200°C, insbesondere im Bereich von 80 bis 180 °C.

Als Hydriergase können beliebige Wasserstoff-haltige Gasgemische, die keine schädlichen Mengen an Katalysatorgiften wie beispielsweise Kohlenmonoxid oder Schwefelwasserstoff enthalten, eingesetzt werden. Die Inertgasbestandteile können beispielsweise Stickstoff oder Methan sein. Bevorzugt wird Wasserstoff in einer Reinheit von größer 95 %, insbesondere größer 98 % eingesetzt.

Die Erfindung wird nachfolgend anhand von Beispielen erläutert. Diese Beispiele zeigen exemplarische Ausführungsformen und sind nicht einschränkend zu verstehen.

Beispiel 1

Das erfindungsgemäße Verfahren wurde in einer technischen Großanlage getestet. Vorher wurde eine Kernhydrierung von Diisononylphthalat (DINP) durchgeführt. Der für die Hydrierung notwendige Wasserstoff wurde aus dem vorhandenen Werksnetz entnommen und mit einem Verdichter auf den erforderlichen Reaktionsdruck verdichtet. Die Hydrierung erfolgt im ersten Reaktor unter einem Reaktionsdruck von ca. 100 bar und einer Reaktionstemperatur von ca. 110 °C an einem als Festbett angeordneten Katalysator (1% Ru auf $TiO_2$ als Trägermaterial). Das DINP wurde im Reaktor bis zu einer Restkonzentration von ca. 10 % umgesetzt. Das Verfahren wird mit zwei Reaktoren durchgeführt, einem Hauptreaktor und einem Nachreaktor. Der Hauptreaktor wird als adiabatischer Kreislaufreaktor betrieben. Die durch die exotherme Hydrierung entstehende Reaktionswärme wird durch Umwälzen eines großen Kreislaufstromes mit einer Pumpe vom Sumpf des Hauptreaktors zurück zum Hauptreaktorkopf über einen Luftkühler abgeführt. Hinter dem Luftkühler wird der Eduktstrom in den Kreislaufstrom eindosiert und vor dem Eintritt in den Hauptreaktor die gewünschte Temperatur durch Regelung der Kreislaufstromtemperatur eingestellt. Ein Austrag aus dem Kreislaufstrom des Hauptreaktors wird zum Nachreaktor geleitet. Die restlichen ca. 10% DINP im Austragsstrom des Hauptreaktors werden im Nachreaktor bei ähnlichen Reaktionsbedingungen wie im Hauptreaktor bis auf < 100 ppm hydriert.

Anschließend wurden die Reaktoren zunächst inertisiert. Dazu wurden die Reaktoren bis auf ca. 3 bar entspannt und danach 3 Mal mit Stickstoff auf ca. 20 bar aufgedrückt und wieder entspannt, um restlichen Wasserstoff aus den Reaktoren zu verdrängen.

Danach erfolgte die Regenerierung. Die Regenerierung wurde bei einer Reaktortemperatur von ca. 60 bis 90°C im Zulauf gestartet. Dazu wurden Hauptreaktor und Nachreaktor separat mit Stickstoff gespült. Die Stickstoffmenge wurde so eingestellt, dass der Druckverlust über die jeweiligen Reaktoren < 0,1 bar war, dies entsprach ungefähr 5 Nm3 pro Stunde und pro $m^3$ Katalysatormaterial für den Hauptreaktor sowie ungefähr 15 $Nm^3$ pro Stunde und pro $m^3$ Katalysatormaterial für den Nachreaktor. Nach 3 bis 6 Stunden wurde in 4 Schritten über 21 Stunden hinweg schrittweise Luft zugegeben, bis der Anteil von Luft am Gasstrom etwa 50 % des Volumenstroms betrug. Anschließend wurde, bei Temperaturen im Reaktor im Bereich von 15 bis 70 °C, die Zugabe von Stickstoff in 2 Schritten über 7 Stunden hinweg entsprechend reduziert bis die Reaktoren nur mit Luft durchströmt werden. Bei allen Schritten wurde darauf geachtet, dass die Temperatur im Reaktor nicht um 10 °C gegenüber der Zulauftemperatur anstieg, um ein Abbrennen es Katalysators durch den Luftsauerstoff zur vermeiden. Die Durchströmung mit Luft wurde noch mehrere Tage fortgeführt.

Vor und nach der Reaktion wurde die Geschwindigkeitskonstante der Hydrierung ermittelt und daraus die Aktivität des Katalysators vorher und nachher berechnet. Die Ermittlung der Geschwindigkeitskonstante erfolgte anhand der verschiedenen Messdaten aus der Anlage mit der Simulationssoftware Aspen Plus® der Firma AspenTech, wobei die Vergleichbarkeit der Daten durch eine definierte Zulaufmenge sichergestellt wurde.

Im Sinne dieser Anmeldung wird die Katalysatoraktivität als Relation aus der ermittelten Geschwindigkeitskonstante ($k_{aktuell}$) und der theoretischen Geschwindigkeitskonstante ($k_{theoretisch}$) bestimmt. Die Katalysatoraktivität wird nach folgender Formel berechnet:

$$Katalysatoraktivit\ddot{a}t = \frac{k_{aktuell}}{k_{theoretisch}} \cdot 100\%$$

Die Ergebnisse der Versuche sind in der Tabelle 1 gezeigt.

Tabelle 1: Ermittelte Katalysatoraktivitäten nach Regenerierung

| Katalysatoraktivität | Regenerierung | |
|---|---|---|
| | vor | nach |
| Hauptreaktor | 60,6% | 95,3% |
| Nachreaktor | 15,5 % | 38,9% |

Es zeigt sich, dass die erfindungsgemäße Regenerierung zu deutlich höheren Katalysatoraktivitäten führt.

**Patentansprüche**

1. Verfahren zur Regenerierung eines für die Kernhydrierung eines Aromaten, vorzugsweise zur Kernhydrierung eines aromatischen Esters, eingesetzten Katalysators in mindestens einem Reaktor, wobei das Verfahren die folgenden Schritte umfasst:

  ▪ Spülen des mindestens einen Reaktors, in dem der eingesetzte Katalysator vorliegt, mit einem Inertgas, wobei das Spülen mit einem Inertgas, vorzugsweise mit Stickstoff bei einer Temperatur von 50 bis 100 °C gestartet wird; gefolgt von
  ▪ einer schrittweisen Zugabe von Luft zum Inertgas während des Spülens des Reaktors bis der Anteil der Luft an dem Gesamtvolumen zwischen 10 und 90 Vol.-% liegt; und anschließend
  ▪ einer Reduzierung der Inertgasmenge, vorzugsweise der Stickstoffmenge, während des Spülens des Reaktors bis der Reaktor nur noch von Luft durchströmt wird,
  wobei die Temperatur im Reaktor während der Regenerierung maximal 10°C höher ist als die Temperatur am Zulauf des Reaktors für den Aromaten, vorzugsweise den aromatischen Ester, bei der Hydrierung des Aromaten, vorzugsweise des aromatischen Esters,
  wobei der Katalysator mindestens ein Übergangsmetall, welches aus der Gruppe, bestehend aus Eisen, Ruthenium, Nickel, Rhodium, Platin, Palladium oder Mischungen davon, ausgewählt wird auf einem Trägermaterial, welches aus der Gruppe, bestehend aus Aktivkohle, Siliciumcarbid, Aluminiumoxid, Siliciumdioxid, Alumosilikat, Titandioxid, Zirkoniumdioxid, Magnesiumoxid, Zinkoxid oder Mischungen davon, ausgewählt wird umfasst.

2. Verfahren nach Anspruch 1, wobei während der schrittweisen Zugabe von Luft und/oder während der Reduzierung der Inertgasmenge die Temperatur in dem mindestens einen Reaktor überwacht wird.

3. Verfahren nach Anspruch 2, wobei die Temperatur während der schrittweisen Zugabe von Luft maximal 20 °C, vorzugsweise maximal 15 °C höher ist als zu Beginn des jeweiligen Schrittes der schrittweisen Zugabe von Luft.

4. Verfahren nach Anspruch 2 oder 3, wobei die (weitere) Zugabe von Luft zumindest zeitweise abgestellt wird, wenn die Temperatur während der schrittweisen Zugabe von Luft 20 °C, vorzugsweise 15 °C oder mehr über der Temperatur zu Beginn des jeweiligen Schrittes der schrittweisen Zugabe von Luft liegt.

5. Verfahren nach Anspruch 4, wobei während des Abstellens Inertgas zudosiert wird.

6. Verfahren nach einem der vorhergehenden Ansprüche, wobei vor dem Spülen des Reaktors mit Stickstoff die folgenden Schritte durchgeführt werden:

   • Entspannen des mindestens einen Reaktors auf einen Druck von weniger als 10 bar, vorzugsweise weniger als 7,5 bar, besonders bevorzugt weniger als 5 bar;
   • Verdrängen des restlichen Wasserstoffs aus dem mindestens einen Reaktor durch Aufdrücken eines Inertgases, vorzugsweise von Stickstoff auf einen Druck von mehr als 10 bar, vorzugsweise mehr als 12.5 bar, besonders bevorzugt mehr als 15 bar und anschließendes Entspannen des mindestens einen Reaktors auf einen Druck von weniger als 10 bar, vorzugsweise weniger als 7,5 bar, besonders bevorzugt weniger als 5 bar.

7. Verfahren nach Anspruch 6, wobei das Aufdrücken eines Inertgases, vorzugsweise von Stickstoff und das anschließende Entspannen mindestens zweimal durchgeführt wird.

8. Verfahren nach einem der vorherigen Ansprüche, wobei die Dauer der gesamten Regenerierung, also die Durchführung aller Schritte, vorzugsweise mindestens 24 Stunden, besonders bevorzugt mindestens 3 Tage beträgt.

9. Verfahren nach einem der vorhergehenden Ansprüche, wobei das Inertgas aus der Gruppe, bestehend aus Stickstoff, Helium, Neon, Argon, Kohlendioxid und Mischungen daraus, ausgewählt wird.

10. Verfahren nach einem der vorhergehenden Ansprüche, wobei der Aromat ein aromatischer Ester ist.

11. Verfahren nach Anspruch 10, wobei der aromatische Ester ein Ester einer Benzolcarbonsäure, einer Benzoldicarbonsäure, einer Benzoltricarbonsäure oder einer Benzoltetracarbonsäure, vorzugsweise ein Ester der Benzoldicarbonsäure oder der Benzoltricarbonsäure, besonders bevorzugt ein Ester der Benzoldicarbonsäure ist.

12. Verfahren nach Anspruch 10 oder 11, wobei der aromatische Ester ein C8- bis C10-Alkylester der Phthalsäure oder ein C8- bis C10-Alkylester der Terephthalsäure ist.

Europäisches
Patentamt

European
Patent Office

Office européen
des brevets

## EUROPÄISCHER RECHERCHENBERICHT

**Nummer der Anmeldung**

**EP 21 21 4724**

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2011/144398 A1 (MIRK DANIELA [DE] ET AL) 16. Juni 2011 (2011-06-16) | 1 | INV. B01J38/04 C07C15/00 |
| Y | * Zusammenfassung * * Absätze [0081] – [0083], [0108] – [0128]; Ansprüche 1,6-22; Beispiel 3 * ----- | 1-12 | B01J23/96 B01J23/94 |
| Y | WO 2008/015135 A2 (BASF AG [DE]; HENKELMANN JOCHEM [DE] ET AL.) 7. Februar 2008 (2008-02-07) * Zusammenfassung * * Seite 3, Zeilen 11-28 * * Seite 11, Zeile 40 – Seite 14, Zeile 21 * * Seite 19, Zeile 19 – Seite 22, Zeile 2 * * Ansprüche 1-14 * ----- | 1-12 | |

**RECHERCHIERTE SACHGEBIETE (IPC)**

B01J
C07C

Der vorliegende Recherchenbericht wurde für alle Patentansprüche erstellt

| Recherchenort | Abschlußdatum der Recherche | Prüfer |
|---|---|---|
| München | 30. Mai 2022 | Fischbach, Malaika |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument
.............................................................................
& : Mitglied der gleichen Patentfamilie, übereinstimmendes
    Dokument

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 21 21 4724

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

30-05-2022

| Im Recherchenbericht angeführtes Patentdokument | Datum der Veröffentlichung | Mitglied(er) der Patentfamilie | | Datum der Veröffentlichung |
|---|---|---|---|---|
| US 2011144398 A1 | 16-06-2011 | CN | 102652037 A | 29-08-2012 |
| | | EP | 2509712 A2 | 17-10-2012 |
| | | US | 2011144398 A1 | 16-06-2011 |
| | | WO | 2011069933 A2 | 16-06-2011 |
| WO 2008015135 A2 | 07-02-2008 | AT | 491515 T | 15-01-2011 |
| | | CN | 101522301 A | 02-09-2009 |
| | | EP | 2049254 A2 | 22-04-2009 |
| | | JP | 5044649 B2 | 10-10-2012 |
| | | JP | 2009545554 A | 24-12-2009 |
| | | KR | 20090037902 A | 16-04-2009 |
| | | MY | 145414 A | 15-02-2012 |
| | | US | 2009305869 A1 | 10-12-2009 |
| | | WO | 2008015135 A2 | 07-02-2008 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2008015103 A1
- WO 03103830 A1